# EUROPÄISCHE PATENTSCHRIFT

(11) **EP 0 606 065 B1**
(45) Veröffentlichungstag und Bekanntmachung des Hinweises auf die Patenterteilung: **25.08.1999**
(21) Anmeldenummer: 94100048.1
(22) Anmeldetag: 04.01.1994
(51) Int. Cl.: C07C 45/61, C07C 41/48, C07C 303/40, C07C 67/30, C07C 253/30, C07D 257/04, C07C 311/51, C07C 69/767, C07C 255/56, C07C 311/58, C07C 311/09

(54) **Verfahren zur Herstellung von Biphenylderivaten**
Method for the preparation of biphenyl derivatives
Procédé pour la préparation de dérivés de biphényle

(30) Priorität: 06.01.1993 DE 4300137
(43) Veröffentlichungstag der Anmeldung: 13.07.1994
(73) Patentinhaber: HOECHST AKTIENGESELLSCHAFT, 65929 Frankfurt am Main (DE)
(72) Erfinder: Wagner, Adalbert, Dr., D-65795 Hattersheim/Main (DE); Bhatnagar, Neerja, Dr., F-91600 Savigny Sur Orge (FR); Buendia, Jean, Dr., F-94170 Le Perreux Sur Marne (FR); Griffoul, Christine, Dr., F-93110 Rosny Sous Bois (FR)

(56) Entgegenhaltungen:
- EP-A- 0 424 317
- EP-A- 0 449 699
- EP-A- 0 499 415
- EP-A- 0 550 313
- DE-A- 2 362 589
- FR-A- 2 632 952
- US-A- 5 130 439
- SYNTHETIC COMMUNICATIONS, Bd. 11, Nr. 7 , 1981 New York, US, Seiten 513 - 519 N. MIYAURA, ET AL.: 'The palladium- catalysed cross-coupling reaction of phenylboronic acid with haloarenes in the presence of bases'
- ORGANOMETALLICS, Bd. 3, Nr. 7 , Juli 1984 Washington, DC, US, Seiten 1261 - 1263 R.B. MILLER, ET AL.: 'Stoichiometric synthesis of unsymetrical mononitro- biphenyls via the palladium-catalysed cross-coupling of arylboronic acids with aryl bromides'
- JOURNAL OF MEDICINAL CHEMISTRY, Bd. 35, Nr. 25 , 11. Dezember 1992 Washington, DC, US, Seiten 4751 - 4763 K.S. ATWAL, ET AL.: 'Dihydropyrimidine angiotensin II receptor antagonists'
- JOURNAL OF MEDICINAL CHEMISTRY, Bd. 35, Nr. 22 , 30. Oktober 1992 Washington, DC, US, Seiten 4027 - 4038 R.H. BRADBURY, ET AL.: 'New nonpeptide angiotensin II receptor antagonists. 2. Synthesis, biological properties, and structure-activity relationships of 2-alkyl-4-(biphenylylmethoxy)quinoline derivatives'
- JOURNAL OF THE CHEMICAL SOCIETY, PERKIN TRANSACTIONS 1, Nr. 6 , Juni 1978 Letchworth, GB, Seiten 653 - 657 S.A. GLOVER, ET AL.: 'N-Iodo-amides: cyclisation of substituted biphenyl-2- carboxamides'
- JOURNAL OF CHROMATOGRAPHY, Bd. 384 , 1987 Amsterdam, NL, Seiten 349 - 356 J.J. SABATKA, ET AL.: 'Measurement of lipophilicity by high-performance liquid chromatography. Comparison with calculated lipophilicity values'

## Beschreibung

Die Erfindung betrifft ein Verfahren zur Herstellung von substituierten Biphenylderivaten sowie Zwischenprodukte zu deren Herstellung.

Bei der Herstellung von Wirkstoffen wie beispielsweise Arzneimitteln für Herz-Kreislauf-Erkrankungen haben sich Biphenylderivate als wichtige Zwischenprodukte im Herstellverfahren ergeben. Beispielsweise wird in EP-A 503 162 die Herstellung von blutdrucksenkenden Präparaten beschrieben, welche als Wirkstoff Verbindungen vom Angiotensin II-Rezeptorantagonist-Typ enthalten, die ein speziell substituiertes Biphenylsystem aufweisen.

Es wurden bereits verschiedene Herstellverfahren für substituierte Biphenylderivate beschrieben, beispielsweise lassen sich Phenylboronsäurederivate mit Arylhalogeniden unter Verwendung von Übergangsmetallkatalysatoren, beispielsweise Palladium, koppeln. Entsprechende Reaktionen wurden von R.B. Miller et al. in Organometallics 1984, 3, 1261 oder von A. Zuzuki et al. in Synthetic Commun. 11(7), 513 (1981) beschrieben. In US 5,130,439 wird ein Herstellverfahren für Tetrazolylsubstituierte Biphenylderivate beschrieben, nachdem ein entsprechend substituiertes Phenylboronsäurederivat mit einer gegebenenfalls durch eine Formylgruppe substituierten Phenylhalogenverbindung umgesetzt wird.

Die Erfindung bezieht sich auf ein Verfahren zur Herstellung von Biphenylderivaten der allgemeinen Formel (I) 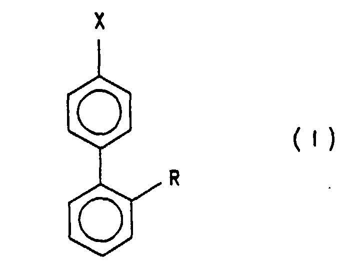 worin
- X: -CHO oder -CH(OR¹)OR² bedeutet,
- R¹, R² =: unabhängig voneinander (C₁-C₆)-Alkyl oder R¹ und R² zusammen eine Alkylengruppe -(CH₂)ₙ- bedeuten, wobei n = 2, 3, 4 oder 5 bedeutet, und
- R: -SO₂NH-COOR³, -SO₂NH-CO-NHR³, -SO₂NH-SO₂-R³ oder -SO₂NR⁴ bedeutet, wobei
- R³: Wasserstoff, (C₁-C₆)-Alkyl, (C₃-C₆)-Cycloalkyl oder (C₁-C₆)-Alkyl-(C₃-C₆)-cycloalkyl bedeutet, und
- R⁴: eine Gruppe =CH-N(CH₃)₂ bedeutet.

Bei dem erfindungsgemäßen Verfahren werden ausgehend von bekannten, gegebenenfalls geschützten Formylbenzolhalogeniden, beispielsweise den Bromiden oder Jodiden, über eine Grignard-Reaktion Boronsäurederivate der Formel (II) hergestellt (siehe z. B. H. Feulner et al.; Chemische Berichte 123 (1990) 1841 bis 1843), wobei die

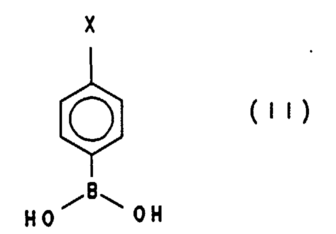

Gruppe X in Formel (II) für eine CHO-Gruppierung oder für eine entsprechend geschützte Formylgruppe, beipielsweise ein Acetal steht. Die Verbindungen der allgemeinen Formel (II) werden dann durch Kopplung mit substituierten Phenylhalogenverbindungen der Formel (III), die nach bekannten Methoden gewonnen werden können, zu den Biphenylverbindungen der Formel (I) umgesetzt, 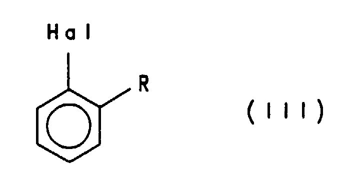 wobei als Halogengruppierung (Hal) für die Verbindungen der allgemeinen Formel (III) Jodide, in Frage kommen, und R wie oben definiert ist.

Statt der Boronsäurederivate der Formel (II) können auch die entsprechenden Boronsäureester für das erfindungsgemäße Verfahren verwendet werden, die beispielsweise ausgehend von den entsprechenden Brommethylbenzolboronsäurederivaten synthetisiert werden können.

Die Verknüpfung der beiden Phenylderivate der Formeln (II) und (III) zur entsprechenden Biphenylverbindung kann unter Verwendung eines Katalysators, vorzugsweise eines Palladiumkatalysators, durchgeführt werden.
Die Reaktionsbedingungen können je nach Reaktivität der Ausgangsstoffe variiert werden, bevorzugt wird ein Temperaturbereich von etwa 20°C bis 150°C und ein Druck von 1 bar bis 5 bar angewandt. Als Lösungsmittel kommen z. B. Gemische aus Benzol oder Toluol mit Alkoholen, insbesonders Ethanol, in Frage.

Die Herstellung der geschützten Formylgruppierung und die Bereitstellung der Grignard-Reagenzien erfolgt nach gängigen Methoden.

Die Erfindung betrifft auch die Verbindungen der allgemeinen Formel (III) als solche, wobei Verbindungen bevorzugt sind, in denen R gleich SO₂NH-COOR³, SO₂NH-CO-NHR³ oder SO₂NH-SO₂R³ bedeutet, wobei SO₂NHCONHR³ besonders bevorzugt ist.

Die Erfindung wird durch die nachfolgenden Beispiele näher erläutert.

### Referenzbeispiel

### Verfahren zur Herstellung von 4-Formyl-2'-N,N-dimethylaminoformyl-biphenylsulfonamid

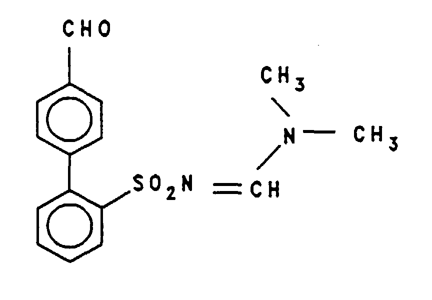

### 1a) 4-Brombenzaldehyd-diethylacetal

Zu 2,7 g Ammoniumnitrat in 65 ml (1,1 Mol) wasserfreiem Ethanol werden 100 g (0,54 Mol) geschmolzener 4-Brombenzaldehyd und 90 ml (0,54 Mol) Orthoameisensäuretriethylester zugegeben. Nach 18 Stunden bei Raumtemperatur wird abfiltriert und mit Piperidin alkalisch gestellt (∼pH 10). Die Titelverbindung erhält man durch Destillation im Vakuum.
- Ausbeute:: 90 %
- Siedepunkt:: (bei 0,05 Torr) = 110 bis 115°C.

### 1b) 4-Formylbenzolboronsäure

Unter Argonatmosphäre werden 3,65 g Magnesiumspäne mit 15 ml wasserfreiem THF überschichtet und mit 0,5 ml 1,2-Dibromethan versetzt. Leichtes Erwärmen führt zu einer heftigen Reaktion. Nach dem Abklingen wird mit einer Pipette das Lösungsmittel abpipettiert, mit 40 ml wasserfreiem THF versetzt und 1/3 einer Lösung von 32 g des Produkts 1 a) in 30 ml wasserfreiem THF zugesetzt. Die Reaktion wird mit Red-Al und durch Erwärmen gestartet. Der Rest des Produktes aus 1 a) wird nun innerhalb von 35 min zugetropft. Nach beendetem Zutropfen wird noch 1 h unter Rückfluß gekocht.

Das Grignardprodukt wird nun zu einer auf -68°C gekühlten Lösung von 33,5 ml Tributylborat unter Argonatmosphäre in 50 ml THF zugetropft. Nach 30 min wird die Kühlung entfernt. Der Ansatz rührt nun 1 h bei RT. Nun wird eingeengt, das honigfarbene Öl wird in 100 ml Ether aufgenommen und es werden 80 ml eiskalte H₂SO₄ (1M) zugesetzt. Die Etherphase wird abgetrennt und noch zweimal mit 50 ml Ether extrahiert, eingeengt und 30 %ige (6N) KOH zugesetzt bis alkalische Reaktion (pH 14) eintritt. 70 ml H₂O werden zugesetzt und im Hochvakuum wird bei 35 bis 40°C das Butanol azetrop abgezogen. Dieser Vorgang wird nochmals mit 50 ml H₂O wiederholt. Der Rückstand wird mit 1M H₂SO₄ sauer gestellt (pH 1) und 30 min gekocht. Durch Filtration erhält man die Titelverbindung als hellgelben Feststoff. Smp = 255 bis 260°C

### 1c) 4-Formyl-2'-N,N-dimethylaminoformylbiphenylsulfonamid

Zu 7 g (0,024 Mol) 2-Brom-N,N-dimethylaminoformylbenzosulfonamid und 0,7 g Triphenylphosphin (0,1 Äquivalente) in 100 ml Toluol werden 5,7 g Natriumcarbonat (2 Äquivalente) in 30 ml H₂O warm dazugegeben. Es wird gut mit Argon gespült und bei 60°C 0,3 g Palladiumacetat (0,05 Äquivalente) im Argongegenstrom zugesetzt. Nach 10 Minuten werden zu der inzwischen schwarzbraunen Reaktionslösung 4 g der Verbindung aus 1 b (1,1 Äquivalente) in 70 ml Ethanol im Argongegenstrom zugeschüttet. Der Ansatz wird anschließend auf Siedetemperatur erhitzt und 3 1/2 Stunden unter Rückfluß gekocht. Nach Abkühlen wird das Lösungsmittel im Vakuum entfernt. Der Rückstand wird in 150 ml Ethylacetat aufgenommen und 5 x mit gesättigter Natriumcarbonatlösung gewaschen. Die organische Phase wird mit Magnesiumsulfat getrocknet und über eine Schicht Kieselgur filtriert. Nach Entfernen des Lösungsmittels im Vakuum fallen 8 g der Titelverbindung als braune, teilweise kristalline, Rohsubstanz an. Diese kann durch Aufkochen in ca. 30 ml Ethylacetat gereinigt werden.
- Ausbeute:: 83 %

R_{f} = 0.4 (E/H 2/1); MS (M + 1) = 317; Smp.: 161°C

Beispiel 1 beschreibt die Darstellung von Zwischenprodukten der allgemeinen Formel III.

### Beispiel 1:

### Verfahren zur Herstellung von 2-Jodbenzol-ṅ-Propylsulfonylharnstoff

### 1a) 2-Jodbenzolsulfonamid

3,5 g 2-Aminobenzolsulfonamid in 25 ml konz. H₂SO₄ (98%) werden auf 60°C erwärmt bis sich eine klare Lösung einstellt und anschließend durch Zugabe von 20 g Eis auf 0°C abgekühlt. 1,45 g NaNO₂ (gelöst in 4ml Wasser) werden vorsichtig tropfenweise hinzugefügt, so daß die Temperatur 5 bis 6°C nicht überschreitet. Anschließend wird das Reaktionsgemisch bei 5 bis 6°C für 3 Stunden gerührt. Nach tropfenweiser Zugabe von 3,75g Kaliumjodid (gelöst in 25 ml Wasser) wird die enstandene rote Mischung erneut für 18 Stunden gerührt. Nach Zugabe von 50 ml Wasser wird der auftretende Niederschlag abfiltriert und mehrmals mit Wasser gewaschen. Die erhaltene Festsubstanz wird in Ethylacetat gelöst, einmal mit 0,2 N Natriumthiosulfatlösung und zweimal mit Wasser gewaschen und das Lösungsmittel abgezogen. Als Ausbeute werden 4 g einer leicht gelblichen Substanz erhalten (62% Ausbeute). Smp: 197-198°C; IR (nujol): 3360, 3255, 1562 cm⁻¹; MS (M⁺): 283

### 1b) 2-Jodbenzol-n-Propylsulfonylharnstoff

3,92 g festes K₂CO₃ werden zu einer gerührten Lösung von 2-Jodbenzolsulfonamid (4g in 40 ml Aceton) aufeinmal hinzugegeben und unter Stickstoffatmosphäre unter Rückfluß gekocht. n-Propylisocyanat wird zum erhitzten Reaktionsansatz zugetropft, der anschließend 2h unter Rückfluß gekocht, auf Zimmertemperatur abgekühlt und zur Trockenheit eingeengt wird. Nach Zugabe von 200 ml Wasser wird der gekühlte Reaktionsansatz mit 2N HCl auf pH 4 eingestellt und filtriert. Der Niederschlag wird aus Aceton/Isopropylether umkristallisiert. Die Ausbeute der Titelverbindung beträgt 4,1 g.
Smp: 211-212°C, IR (nujol): 3406, 3368, 1715, 1565, 1539 cm⁻¹; MS (M⁺): 368

Die ¹H-NMR-Daten der Titelverbindungen des Referenzbeispich sowie der Beispiele 1a und 1b sind in Tabelle 2 zusammengefaßt.

**Tabelle 2**

| Referenzbeispiel |
|---|
| (DMSO-d₆) d = 2,68 (s, 3 H); d = 2,72 (s, 3 H); d = 7,2 (s, 1 H); d = 7,25 bis 7,35 (m, 1 H); d = 7,45 bis 7,75 (m, 4 H); d = 7,95 (d, J = 8 Hz, 1 H); d = 8,05 bis 8,15 (m, 2 H); d = 10,1 (s, 1 H). |

1a. (CDCl₃) 5,17 (s, l, NH₂); 7,23 bis 7,52 (td Aromaten 2H); 8,08 bis 8,20 (dd, Aromaten 2H)
1b: (CDCl₃) 0,82 (t, J = 7,5 CH₃, 3H); 1,45 (m, CH₂, 2H; 3,14 (m, CH₂, 2H); 6,39 (t, CONH, 1H); 7,29 (dt, J = 1,5 Aromaten); 7,54 (td, J = 8,15 Aromaten); 8,13 (m, Aromaten) 7,59 (s, SO₂NH, 1H).
Abkürzungen:

| | |
|---|---|
| E | Ethylacetat |
| H | n-Heptan |
| Red-Al | Natrium-dihydrido-bis-(2-methoxy-aethoxy)-aluminat |
| THF | Tetrahydrofuran |

## Patentansprüche

1. Verfahren zur Herstellung einer Verbindung der allgemeinen Formel (I), 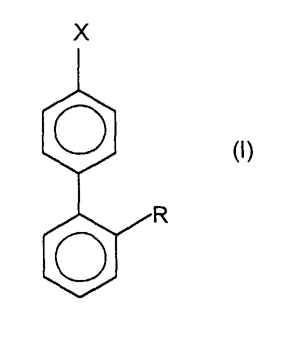 worin
X -CHO oder -CH(OR¹ )OR² bedeutet, wobei
R¹, R² unabhängig voneinander (C₁-C₆)-Alkyl oder R¹ und R² zusammen eine Alkylengruppe -(CH₂)ₙ- bedeuten wobei n = 2, 3, 4 oder 5 bedeutet, und
R -SO₂NH-COOR³, -SO₂NH-CO-NHR³, -SO₂NH-SO₂-R³ oder -SO₂NR⁴ bedeutet, wobei
R³ Wasserstoff, (C₁-C₆)-Alkyl, (C₃-C₆)-Cycloalkyl oder (C₁-C₆)-Alkyl-(C₃-C₆)-cycloalkyl bedeutet, und
R⁴ eine Gruppe =CH-N(CH₃)₂ bedeutet,
dadurch gekennzeichnet, daß man eine Verbindung der allgemeinen Formel (II) 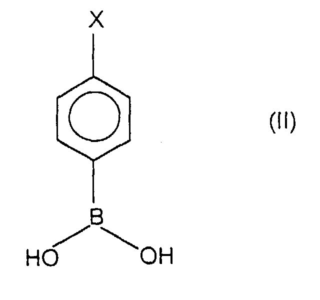 worin X wie oben definiert ist, oder einer entsprechenden Boronsäure ester mit einer substituierten Phenylhalogenverbindung der Formel (III) 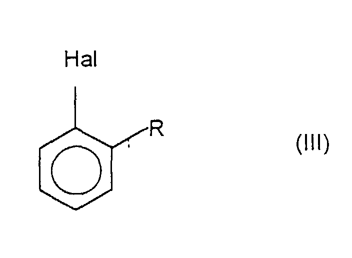 umsetzt, wobei der Substituent Hal für Jod steht und R wie oben definiert ist.

2. Verfahren gemäß Anspruch 1, dadurch gekennzeichnet daß die Kopplung der Verbindung der allgemeinen Formel (II) mit einer Verbindung der allgemeinen Formel (II) unter Einsatz des Übergangsmetallkatalysators Palladium erfolgt.

3. Verbindung der allgemeinen Formel (III) 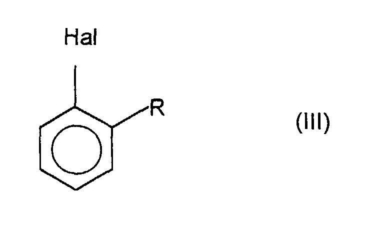 worin die Substituenten folgende Bedeutung haben:
Hal Jod und
R -SO₂NH-COOR³, -SO₂NH-CO-NHR³ oder -SO₂NH-SO₂-R³ bedeutet, wobei
R³ Wasserstoff, (C₁-C₆)-Alkyl, (C₃-C₆)-Cycloalkyl oder (C₁-C₆)-Alkyl-(C₃-C₆)-cycloalkyl bedeutet.

4. Verbindung gemäß Anspruch 3, dadurch gekennzeichnet, daß in Formel (III)
R gleich -SO₂NH-CO-NHR³ ist, wobei
R³ (C₁-C₆)-Alkyl bedeutet.

## Claims

1. A process for the preparation of a compound of the formula (I) 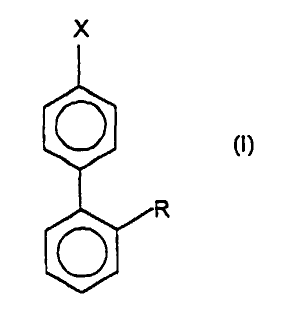 in which
X is -CHO or -CH (OR¹) OR², where
R¹ and R² independently of one another are (C₁-C₆)-alkyl or R¹ and R² together are an alkylene group - (CH₂)ₙ-, where n is 2, 3, 4 or 5, and
R is -SO₂NH-COOR³, -SO₂NH-CO-NHR³, -SO₂NH-SO₂-R³ or -SO₂NR⁴, where
R³ is hydrogen, (C₁-C₆)-alkyl, (C₃-C₆)-cycloalkyl or (C₁-C₆)-alkyl-(C₃-C₆)-cycloalkyl and
R⁴ is a group =CH-N(CH₃)₂,
which comprises reacting a compound of the formula (II) 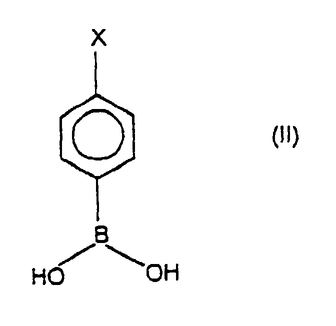 where X is as defined above, or a corresponding boronic acid ester with a substituted phenylhalogen compound of the formula (III) 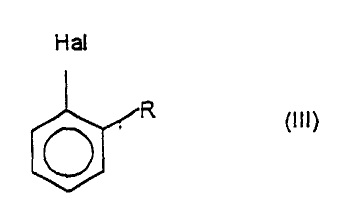 where the substituent Hal is iodine and R is as defined above.

2. The process as claimed in claim 1, wherein the coupling of the compound of the formula (II) to a compound of the formula (III) is carried out using the transition metal catalyst palladium.

3. A compound of the formula (III) 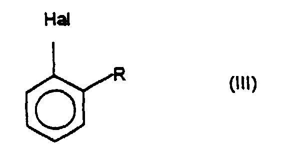 in which the substituents have the following meaning:
Hal is iodine and
R is -SO₂NH-COOR³, -SO₂NH-CO-NHR³ or -SO₂NH-SO₂-R³, where
R³ is hydrogen, (C₁-C₆)-alkyl, (C₃-C₆)-cycloalkyl or (C₁-C₆)-alkyl-(C₃-C₆)-cycloalkyl.

4. A compound as claimed in claim 3, wherein, in formula (III)
R is -SO₂NH-CO-NHR³, where
R³ is (C₁-C₆)-alkyl.

## Revendications

1. Procédé pour la préparation d'un composé de formule générale (I) 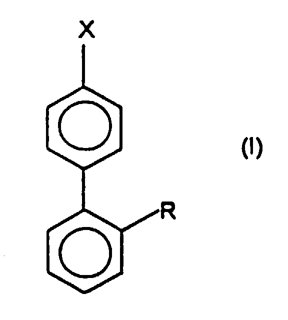 dans laquelle
X représente -CHO ou -CH(OR¹)OR²,
R¹, R² représentent, indépendamment l'un de l'autre, un groupe alkyle en C₁-C₆, ou R¹ et R² forment ensemble un groupe alkylène -(CH₂)ₙ-, n étant égal à 2, 3, 4 ou 5, et
R représente -SO₂NH-COOR³, -SO₂NH-CO-NHR³, -SO₂NH-SO₂ -R ou -SO₂NR⁴,
R³ représentant un atome d'hydrogène ou un groupe alkyle en C₁-C₆, cycloalkyle en C₃-C₆ ou alkyl-
R⁴ représentant le groupe =CH-N(CH₃)₂,
caractérisé en ce que l'on fait réagir un composé de formule générale (II) 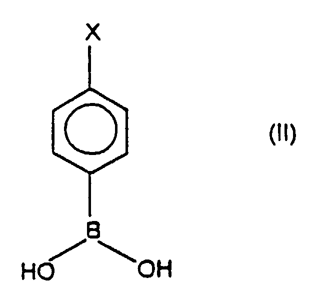 dans laquelle X est tel que défini plus haut, ou un ester d'acide borique correspondant,
avec un composé de type phényle halogéné substitué de formule (III) 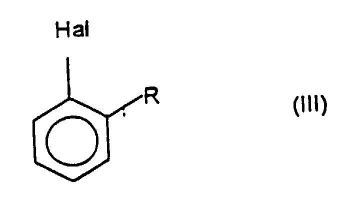 le substituant Hal représentant l'iode et R étant tel que défini plus haut.

2. Procédé selon la revendication 1, caractérisé en ce que le couplage du composé de formule générale (II) avec un composé de formule générale (III) s'effectue avec utilisation du catalyseur de type métal de transition palladium.

3. Composé de formule générale (III) 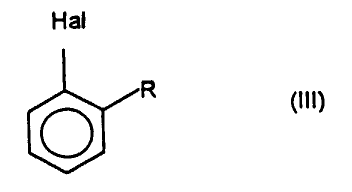 dans laquelle les substituants ont les significations suivantes:
Hal représente l'iode et
R représente -SO₂NH-COOR³, -SO₂NH-CO-NHR³ ou -SO₂NH-SO₂-R³,
R³ représentant un atome d'hydrogène ou un groupe alkyle en C₁-C₆, cycloalkyle en C₃-C₆ ou alkyl-(C₁-C₆)cycloalkyle(C₃-C₆) .

4. Composé selon la revendication 3, caractérisé en ce que, dans la formule (III),
R est -SO₂NH-CO-NHR³,
R³ représentant un groupe alkyle en C₁-C₆.
